# FASCICULE DE BREVET EUROPEEN

(11) **EP 3 625 196 B1**
(45) Date de publication et mention de la délivrance du brevet: **22.12.2021**
(21) Numéro de dépôt: 18723863.9
(22) Date de dépôt: 17.05.2018
(51) Int. Cl.: C07C 7/00, C07C 7/04, C07C 7/11, F25J 3/00, B01D 53/00, C10G 7/00, C10G 70/04, C10G 70/06, C10G 5/06, C07C 7/09, F25J 3/02

(54) **PROCÉDÉ DE RÉCUPÉRATION D'UN COURANT D'HYDROCARBURES EN C2+ DANS UN GAZ RÉSIDUEL DE RAFFINERIE ET INSTALLATION ASSOCIÉE**
VERFAHREN ZUR RÜCKGEWINNUNG EINES STROMS VON C2+-KOHLENWASSERSTOFFEN IN EINEM RAFFINERIERESTGAS UND ZUGEHÖRIGE ANLAGE
METHOD FOR RECOVERING A STREAM OF C2+ HYDROCARBONS IN A RESIDUAL REFINERY GAS AND ASSOCIATED FACILITY

(30) Priorité: 18.05.2017 FR 1754426
(43) Date de publication de la demande: 25.03.2020
(73) Titulaire: Technip France, 92400 Courbevoie (FR)
(72) Inventeur: SIMON, Yvon, 78570 Andresy (FR); DESTOUR, Bruno, 92500 Rueil Malmaison (FR); VALENTE, Marco, 92800 Puteaux (FR)
(74) Mandataire: Lavoix
(86) Numéro de dépôt international: PCT/EP2018/062992
(87) Numéro de publication internationale: WO 2018/211036

(56) Documents cités:
- WO-A2-2014/064172
- FR-A1- 2 957 931
- US-A1- 2004 148 964

## Description

La présente invention concerne un procédé de récupération d'un courant d'hydrocarbures en C2+ dans un gaz résiduel de raffinerie selon la revendication 1.

Un tel procédé est destiné à être mis en œuvre dans une unité de récupération des gaz résiduels de raffinerie pour valoriser le contenu de ces gaz.

D'une manière connue, les gaz de raffinerie sont riches en hydrocarbures légers. Dans certains cas, ils sont envoyés directement vers des réseaux de gaz combustible, étant alors valorisés en tant que combustible. Cependant, certains hydrocarbures légers ont une valeur commerciale significative sur le marché. En particulier, l'éthylène, le propylène, l'hydrogène sont des produits qui peuvent être directement vendus. L'éthane, le propane, les hydrocarbures en C4+ peuvent être également utilisés comme alimentation pour un four de craquage.

La récupération de ces hydrocarbures est donc susceptible d'améliorer la rentabilité d'une raffinerie ou d'un vapocraqueur recevant ces gaz résiduels, intégré ou non à la raffinerie.

Pour récupérer ces hydrocarbures, il est connu par exemple de US 3,320,754 d'effectuer des étapes successives de refroidissement, de condensation et de séparation dans des ballons successifs pour descendre la température du courant de gaz résiduel de raffinerie.

Le liquide collecté dans le dernier ballon est introduit dans une colonne d'élimination du méthane, pour permettre une séparation entre d'un côté, le méthane et les gaz légers, et d'un autre côté les hydrocarbures en C2+.

Les gaz collectés dans le dernier ballon forment un courant d'effluent qui est ensuite réchauffé dans une boîte froide en refroidissant une fraction du gaz résiduel.

Dans une amélioration du procédé décrit dans US 3,320,754, pour améliorer la récupération des hydrocarbures en C2+, le courant d'effluent à la sortie de la boite froide est ensuite détendu dans une turbine de détente dynamique.

Le courant détendu est alors réintroduit dans la boîte froide pour fournir les frigories nécessaires à la condensation partielle du courant gazeux résiduel en amont du dernier ballon, depuis une température sensiblement égale à -100 °C jusqu'à une température de -110°C à -115°C.

Le froid fourni par la turbine de détente dynamique est donc nécessaire pour descendre significativement la température des gaz dans la gamme requise, ce qui minimise les pertes en éthylène et en éthane dans le gaz de tête de la colonne de distillation.

FR 2 957 391 décrit en ce sens un procédé de traitement d'un courant de gaz issu d'une installation de pyrolyse d'hydrocarbures, ce procédé comprenant la mise en relation d'échange thermique avec une fraction détendue du courant intermédiaire de tête pour condenser le courant intermédiaire de tête.

Cependant, la présence d'une turbine de détente dynamique complique la mise en œuvre du procédé et l'installation associée à cette mise en œuvre.

Ainsi, l'installation doit être équipée avec la turbine de détente dynamique, et avec une boîte froide de structure complexe pour mettre en circulation le gaz issu de la turbine de détente dynamique.

Ceci requiert un investissement de départ important, et crée un encombrement significatif dans l'installation. En outre, la turbine de détente dynamique doit subir des maintenances régulières et peut tomber en panne, ce qui limite la fiabilité et la productivité du procédé.

Un but de l'invention est d'obtenir un procédé récupérant de manière très efficace et fiable des produits valorisables dans les gaz résiduels de raffinerie, tout en limitant les coûts d'investissement et d'exploitation du procédé.

À cet effet, l'invention a pour objet un procédé la séparation du flux de tête refroidi comporte l'introduction du flux de tête refroidi dans un absorbeur, et l'injection d'un courant riche en méthane dans l'absorbeur pour mettre en contact le flux de tête refroidi avec le courant riche en méthane.

Le procédé selon l'invention peut comprendre l'une ou plusieurs des caractéristiques des revendications 2 à 10 et suivantes, prise(s) isolément ou suivant toute combinaison techniquement possible :
- le courant riche en méthane est détendu avantageusement dans une vanne de détente statique avant son injection dans l'absorbeur ;
- le courant riche en méthane est au moins partiellement vaporisé dans la vanne de détente dynamique ;
- le ou chaque courant d'effluent est formé à partir du courant de tête et/ou du courant supérieur, sans passage par une vanne de détente statique et/ou par une turbine de détente dynamique ;
- aucune fraction du courant de tête gazeux n'est introduite dans l'absorbeur ;
- le courant de tête gazeux est mélangé au courant supérieur gazeux en aval de l'absorbeur, sans passer par l'absorbeur ;
- la totalité du flux de tête gazeux issu du ballon de flash est introduite dans l'échangeur thermique ;
- la totalité du flux de tête refroidi issu de l'échangeur thermique est introduit dans l'absorbeur.

L'invention sera mieux comprise à la lecture de la description qui va suivre, donnée uniquement à titre d'exemple, et faite en se référant aux dessins annexés, sur lesquels :

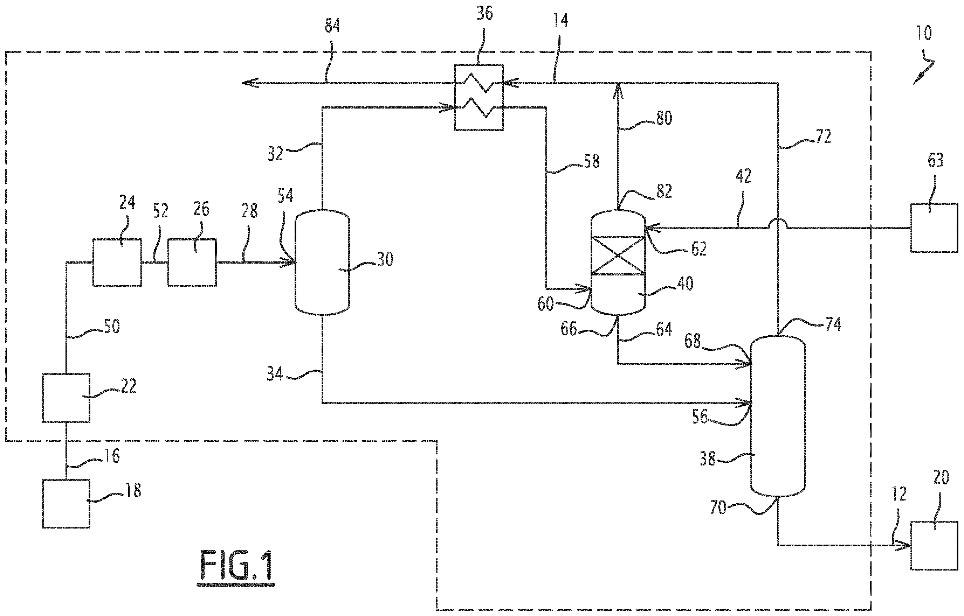

- la figure 1 est un schéma synoptique représentant une première installation destinée à la mise en œuvre d'un procédé selon l'invention.

Une première installation 10 destinée à la récupération d'un courant 12 d'hydrocarbures en C2+, et d'un courant 14 d'effluent à partir d'un gaz résiduel brut 16 de raffinerie par un procédé selon l'invention est illustrée schématiquement sur la figure 1.

L'installation 10 est raccordée en amont à une raffinerie 18 produisant le gaz résiduel 16 brut. L'installation 10 est raccordée en aval à une installation 20 de fractionnement et/ou de récupération d'éthylène.

L'installation 10 comporte une unité 22 de purification destinée à éliminer les contaminants présents dans le gaz résiduel de raffinerie 16, une unité de compression 24, et avantageusement un ou plusieurs étages 26 de refroidissement et de séparation destinés à produire un courant résiduel 28.

L'installation 10 comporte en outre un ballon de flash 30 destiné à recevoir le courant résiduel 28 pour produire un flux de tête 32 gazeux et un flux de pied 34 liquide, un échangeur thermique 36 à deux flux destiné à refroidir le flux de tête gazeux 32 et une colonne de distillation 38.

Selon l'invention, l'installation 10 comporte en outre un absorbeur 40 interposé entre l'échangeur thermique 36 et la colonne de distillation 38, l'absorbeur 40 étant alimenté par un courant riche en méthane 42.

L'unité de purification 22 comporte au moins un étage d'élimination des métaux lourds et des gaz acides, une unité optionnelle d'élimination de l'oxygène, une unité d'élimination de l'eau et une unité d'élimination poussée des impuretés.

L'unité de compression 24 comporte au moins un compresseur, elle peut être localisée en amont ou en aval de l'unité de purification 22.

Chaque étage de refroidissement et de séparation 26 comporte au moins un échangeur thermique destiné à descendre progressivement la température du gaz résiduel pour créer une fraction liquide et une fraction gazeuse, au moins un ballon de flash, destiné à séparer la fraction liquide de la fraction gazeuse, après le refroidissement ainsi qu'éventuellement une colonne de fractionnement dont l'objectif est de limiter dans le flux 28 la teneur en hydrocarbures plus lourds que l'éthane.

Dans tout ce qui suit, les pourcentages sont par défaut des pourcentages molaires. Les pressions sont exprimées en barg.

Les courants décrits dans l'installation sont confondus avec les conduites qui les transportent.

La mise en œuvre du premier procédé selon l'invention va maintenant être décrite.

Initialement, au moins un gaz brut 16 issu de la raffinerie 18 est récupéré. Le gaz brut 16 comprend par exemple entre 1 % molaire et 20 % molaire d'azote, entre 5 % molaire et 60 % molaire d'hydrogène, entre 10 % molaire et 70 % molaire de méthane, entre 5 % molaire et 50 % molaire d'hydrocarbures en C2, entre 0 % molaire et 5 % molaire d'hydrocarbures en C3, et entre 0 % molaire et 4 % molaire d'hydrocarbures en C4+.

Le gaz brut 16 comprend généralement des contaminants, tels que l'oxygène, les oxydes d'azote, l'arsenic, l'ammoniac, le mercure, le monoxyde de carbone, le dioxyde de carbone. Le gaz brut comprend généralement entre 1 et 10% molaire des contaminants listés ci-dessus.

Le gaz brut 16 est introduit dans l'unité de purification 22 pour produire un gaz purifié 50 présentant une teneur en impuretés inférieures à celle du gaz brut, et notamment inférieure à 1% molaire au total.

Le gaz brut purifié 50 est ensuite comprimé dans l'unité de compression 24 jusqu'à une pression supérieure à 10 barg, et comprise notamment entre 10 barg et 30 barg.

Le gaz purifié comprimé 52 issu de l'unité de compression 24 est ensuite introduit dans chaque étage successif de refroidissement et de séparation pour être partiellement condensé et produire le courant résiduel 28.

Le courant résiduel 28 comprend généralement entre 1 % molaire et 25 % molaire d'azote, entre 5% molaire et 70 % molaire d'hydrogène, entre 10 % molaire et 80 % molaire de méthane, entre 5% molaire et 45 % molaire d'hydrocarbures en C2, entre 0 % molaire et 1% molaire d'hydrocarbures en C3, et moins de 1% molaire d'hydrocarbures en C4+.

Il présente une température inférieure à -80°C et comprise avantageusement entre -90 ° C et -100 °C. Il présente une pression supérieure à 10 barg et comprise avantageusement entre 10 barg et 30 barg.

Le courant résiduel 28 est introduit dans le ballon de flash 30 à travers une entrée d'introduction 54 pour y être séparé en le flux gazeux de tête 32 et le flux liquide de pied 34.

Le flux liquide de pied 34 est détendu, avantageusement dans une vanne de détente statique, pour être introduit dans la colonne de distillation 38 au niveau d'une première entrée 56.

La pression du flux liquide de pied 34, prise au niveau de l'entrée 56 est inférieure à 15 barg et est avantageusement comprise entre 8 barg et 12 barg.

Le flux gazeux de tête 32 est introduit dans l'échangeur thermique 36 pour produire, à la sortie de l'échangeur thermique 36, un flux de tête refroidi 58.

Le flux de tête refroidi 58 présente une température inférieure à -90°C et avantageusement comprise entre -100° C et -130°C.

Le flux de tête refroidi 58 est avantageusement au moins partiellement condensé. Il présente une teneur molaire en liquide supérieure à 5% molaire, notamment comprise entre 5 % molaire et 20 % molaire.

Le flux de tête refroidi 58 est ensuite introduit dans l'absorbeur 40 à travers une entrée inférieure 60 située en dessous de l'entrée supérieure 62 d'injection du courant riche en méthane 42.

Le flux de tête 32 circule depuis le ballon de flash 30 à travers l'échangeur thermique 36 et jusqu'à l'absorbeur 40 sans détente statique ou dynamique dans une vanne de détente ou dans une turbine de détente dynamique.

L'absorbeur 40 est une colonne comprenant des plateaux ou/et du garnissage. Il est apte à mettre en contact sans réaction chimique le flux de tête 58 avec le courant riche en méthane 42.

Le courant riche en méthane 42 injecté par l'entrée supérieure 62 présente avantageusement une teneur en méthane supérieure à 95 % molaire. Il présente une température inférieure à -90 °C et notamment comprise entre -95 °C et -130°C.

Le débit molaire du courant riche en méthane 42 est faible. Avantageusement, le débit molaire du courant riche en méthane 42 introduit dans l'absorbeur 40 est inférieur à 25 % du débit molaire du flux de tête 32, pris à la sortie du ballon de flash 30.

Le courant riche en méthane 42 est un courant liquide. Il présente une teneur en liquide supérieure à 70 % molaire, avant détente au travers d'une vanne de détente statique pour former le flux injecté à l'entrée supérieure 62.

Le courant riche en méthane 42 est par exemple produit dans une installation de craquage 63, en particulier dans un vapocraqueur. L'installation de craquage 63 est située au voisinage de l'installation 10, par exemple dans la raffinerie 18

Le courant riche en méthane 42 est fourni à l'absorbeur 40 directement depuis l'installation de craquage 63, sans qu'un passage par un autre équipement de l'installation 10, comme l'échangeur thermique 36, ne change l'objet de la présente invention.

Un courant inférieur liquide 64 est produit dans une sortie inférieure 66 de l'absorbeur 40. Le courant inférieur liquide 64 contient plus de 85% molaire des hydrocarbures en C2+ contenus dans le flux de tête 58.

Le courant inférieur 64 est détendu jusqu'à une pression inférieure à 15 barg et comprise avantageusement entre 8 barg et 12 barg.

Il est alors introduit en reflux dans la colonne de distillation 38 par une entrée supérieure 68 située au-dessus de l'entrée 56 d'introduction du flux de pied 34.

La colonne de distillation 38 opère à une pression inférieure à 15 barg et comprise avantageusement entre 8 barg et 12 barg.

Le courant 12 d'hydrocarbures en C2+ est produit à une sortie de récupération 70 située au pied de la colonne de distillation 38.

Il contient entre 70% molaire et 99% molaire des hydrocarbures en C2+ contenus dans le courant résiduel 28. Le courant 12 d'hydrocarbures en C2+ est ensuite amené jusqu'à l'installation 20 de fractionnement et/ou de récupération de l'éthylène.

Un courant de tête gazeux 72 est récupéré à une sortie d'extraction 74 située en tête de la colonne de distillation 38. Le courant 72 contient plus de 95% molaire du méthane qui est introduit dans la colonne 38.

Le courant de tête gazeux 72 présente une température inférieure à -95°C et notamment comprise entre -100°C et -130°C.

Simultanément, un courant supérieur gazeux 80 est produit à une sortie supérieure 82 située en tête de l'absorbeur 40.

Le courant supérieur 80 est fortement refroidi. Il présente une température inférieure à celle du courant de tête gazeux 72 et à celle du flux de tête 32. La température du courant supérieur est inférieure à -100°C et est notamment comprise entre -110°C et -130°C.

Le courant supérieur 80 est détendu jusqu'à une pression inférieure à 8 barg et comprise entre 2 barg et 6 barg, puis est mélangé dans un piquage formant mélangeur au courant de tête 72 pour produire le courant d'effluent 14.

Le courant d'effluent 14 présente une température inférieure à -95°C et notamment comprise entre -100 °C et -130°C.

Il est alors introduit dans l'échangeur thermique 36 pour entrer en relation d'échange thermique avec le flux de tête 32 et refroidir le flux de tête 32. La différence de température entre le flux de tête refroidi 58 et le flux de tête 32 de part et d'autre de l'échangeur thermique 36 est supérieure à 5°C en valeur absolue.

Le courant d'effluent réchauffé 84 récupéré à la sortie de l'échangeur thermique 36 est ensuite envoyé vers une unité de fractionnement d'hydrogène et/ou vers un réseau de gaz combustible.

Dans un exemple opérationnel de mise en œuvre de l'invention, les conditions opératoires sont les suivantes :

| Courant | Température (°C) | Pression (barg) |
|---|---|---|
| 12 | -36 | 10,1 |
| 32 | -96 | 16,7 |
| 58 | -110 | 16,5 |
| 64 | -111 | 16,5 |
| 72 | -101 | 9,8 |
| 80 | -120 | 16.4 |

Dans le procédé selon l'invention, la puissance thermique nécessaire au refroidissement du flux gazeux de tête 32 est fournie exclusivement par le courant d'effluent 14 provenant de l'absorbeur 40 et de la colonne de distillation 38, sans détente statique ou dynamique de ce courant 14 dans une vanne de détente ou dans une turbine de détente dynamique.

Ainsi, il n'est pas nécessaire de disposer d'une installation de détente dynamique du courant d'effluent 14 à la sortie de l'échangeur thermique 36, ou en amont de celui-ci, pour fournir des frigories nécessaires au refroidissement du flux de tête 32.

L'introduction du courant riche en méthane 42 dans l'absorbeur 40 suffit à produire un courant supérieur gazeux 80 appauvri en C2+ et refroidi qui dispose de la puissance thermique nécessaire au refroidissement. Ainsi, l'échangeur thermique 36 présente une structure très simple, puisqu'il est limité à deux flux.

Le coût d'investissement pour construire l'installation 10 nécessaire à la mise en œuvre du procédé est substantiellement réduit, puisque cette installation 10 ne nécessite pas de turbine de détente dynamique pour produire le froid nécessaire à refroidir le flux de tête 32 aux températures souhaitées. L'installation 10 présente en outre un encombrement plus faible.

La maintenance de l'installation 10 est grandement simplifiée par l'absence d'un élément rotatif clé, et sa fiabilité augmentée.

Ceci est obtenu sans nuire à la récupération des hydrocarbures en C2+, non seulement en régime continu, mais également lors des phases transitoires d'arrêt de l'installation 10.

Dans une variante (non représentée), l'échangeur thermique 36 est un échangeur thermique 36 à trois flux. Un premier courant d'effluent est constitué du courant de tête 72 et un deuxième courant d'effluent est constitué du courant supérieur 80 qui sont introduits séparément dans l'échangeur thermique 36 pour entrer en relation d'échange thermique avec le flux de tête 32. Le flux de tête 32 est refroidi exclusivement par le premier courant d'effluent et par le deuxième courant d'effluent, entre le ballon de flash 30 et l'absorbeur 40.

## Revendications

1. Procédé de récupération d'un courant (12) d'hydrocarbures en C2+ dans un gaz résiduel de raffinerie (16), comprenant les étapes suivantes :
- formation d'un courant résiduel (28) à partir du gaz résiduel de raffinerie (16) ;
- introduction du courant résiduel (28) dans un ballon de flash (30) pour former un flux de tête (32) gazeux et un flux de pied (34) liquide ;
- introduction du flux de pied (34) dans une colonne de distillation (38),
- refroidissement du flux de tête (32) dans un échangeur thermique (36) pour former un flux de tête refroidi (58) ;
- séparation du flux de tête refroidi (58) en un courant inférieur (64) liquide d'alimentation de la colonne de distillation (38) et en un courant supérieur (80) gazeux;
- introduction du courant inférieur (64) dans la colonne de distillation (38) au-dessus du flux de pied (34) ;
- récupération, en pied de la colonne de distillation (38), du courant (12) d'hydrocarbures en C2+ ;
- extraction, en tête de la colonne de distillation (38), d'un courant de tête (72) gazeux ;
- formation d'au moins un courant d'effluent (14) à partir du courant de tête (72) et/ou du courant supérieur (80) ;
- réchauffement du ou de chaque courant d'effluent (14) dans l'échangeur thermique (36) par échange thermique avec le flux de tête (32) ;
**caractérisé en ce que** la séparation du flux de tête refroidi (58) comporte l'introduction du flux de tête refroidi (58) dans un absorbeur (40), et l'injection d'un courant riche en méthane (42) dans l'absorbeur (40) pour mettre en contact le flux de tête refroidi (58) avec le courant riche en méthane (42), la teneur molaire en méthane dans le courant riche en méthane (42) étant supérieure à 90% molaire.

2. Procédé selon la revendication 1, dans lequel le courant riche en méthane (42) est un courant liquide.

3. Procédé selon l'une quelconque des revendications précédentes, dans lequel le courant riche en méthane (42) est formé à partir d'une installation de craquage thermique à la vapeur (63), sans passage par la colonne de distillation (38) ou par le ballon de flash (30).

4. Procédé selon l'une quelconque des revendications précédentes, dans lequel la température du courant résiduel (28) avant son introduction dans le ballon de flash (30) est inférieure à -80°C et est avantageusement comprise entre -90 °C et -100°C.

5. Procédé selon l'une quelconque des revendications précédentes, dans lequel le flux de tête (32) circule depuis le ballon de flash (30) à travers l'échangeur thermique (36) jusqu'à l'absorbeur (40) sans détente statique ou dynamique dans une vanne de détente ou dans une turbine de détente dynamique.

6. Procédé selon l'une quelconque des revendications précédentes, dans lequel l'échangeur thermique (36) est un échangeur thermique (36) à deux flux, le procédé comprenant le mélange du courant de tête (72) et/ou du courant supérieur (80) pour former le courant d'effluent (14), le flux de tête (32) étant refroidi exclusivement par le courant d'effluent (14) entre le ballon de flash (30) et l'absorbeur (40).

7. Procédé selon l'une quelconque des revendications 1 à 5, dans lequel l'échangeur thermique (36) est un échangeur thermique (36) à trois flux, le procédé comprenant la formation d'un premier courant d'effluent à partir du courant de tête (72) et d'un deuxième courant d'effluent à partir du courant supérieur (80), le flux de tête (32) étant refroidi exclusivement par le premier courant d'effluent et par le deuxième courant d'effluent, entre le ballon de flash (30) et l'absorbeur (40).

8. Procédé selon l'une quelconque des revendications précédentes, dans lequel la différence de température entre le flux de tête refroidi (58) et le flux de tête (32) est supérieure à 2°C en valeur absolue.

9. Procédé selon l'une quelconque des revendications précédentes, dans lequel le courant (12) d'hydrocarbures en C2+ contient plus de 90 % molaire des hydrocarbures en C2 contenus dans le courant résiduel (28).

10. Procédé selon l'une quelconque des revendications précédentes comportant une étape préalable de purification et/ou des étapes successives de refroidissement et de séparation du gaz résiduel de raffinerie (16).

## Patentansprüche

1. Verfahren zur Rückgewinnung eines Stroms (12) aus C2+-Kohlenwasserstoffen in einem Raffinerieabgas (16), umfassend die folgenden Schritte:
- Bilden eines Reststroms (28) aus dem Raffinerieabgas (16);
- Einleiten des Reststroms (28) in einen Entspannungskolben (30), um einen gasförmigen Kopfstrom (32) und einen flüssigen Bodenstrom (34) zu bilden;
- Einleiten des Bodenstroms (34) in eine Destillationssäule (38);
- Abkühlen des Kopfstroms (32) in einem Wärmetauscher (36), um einen abgekühlten Kopfstrom (58) zu bilden;
- Aufteilen des abgekühlten Kopfstroms (58) in einen flüssigen unteren Strom (64) zum Speisen der Destillationssäule (38) und einen gasförmigen oberen Strom (80);
- Einleiten des unteren Stroms (64) in die Destillationssäule (38) oberhalb des Bodenstroms (34);
- Rückgewinnung, am Boden der Destillationssäule (38), des Stroms (12) aus C2+-Kohlenwasserstoffen;
- Entnehmen, am Kopf der Destillationssäule (38), eines gasförmigen Kopfstroms (72);
- Bilden von mindestens einem Abflussstrom (14) aus dem Kopfstrom (72) und/oder dem oberen Strom (80);
- Erwärmung des oder jedes Abflussstroms (14) in dem Wärmetauscher (36) durch Wärmeaustausch mit dem Kopfstrom (32);
**dadurch gekennzeichnet, dass** die Aufteilung des abgekühlten Kopfstroms (58) die Einleitung des abgekühlten Kopfstroms (58) in einen Absorber (40) und das Einspritzen eines methanreichen Stroms (42) in den Absorber (40) umfasst, um den abgekühlten Kopfstrom (58) mit dem methanreichen Strom (42) in Kontakt zu bringen, wobei der molare Gehalt an Methan in dem methanreichen Strom (42) größer ist als 90 Mol-%.

2. Verfahren nach Anspruch 1, wobei der methanreiche Strom (42) ein flüssiger Strom ist.

3. Verfahren nach einem der vorherigen Ansprüche, wobei der methanreiche Strom (42) aus einer thermischen Dampfspaltanlage (63) gebildet wird, ohne die Destillationssäule (38) oder den Entspannungskolben (30) zu durchlaufen.

4. Verfahren nach einem der vorherigen Ansprüche, wobei die Temperatur des Reststroms (28) vor seiner Einleitung in den Entspannungskolben (30) niedriger ist als - 80 °C und vorteilhafterweise zwischen -90 °C und -100 °C ist.

5. Verfahren nach einem der vorherigen Ansprüche, wobei der Kopfstrom (32) ohne statische oder dynamische Expansion in einem Expansionsventil oder in einer dynamischen Expansionsturbine von dem Entspannungskolben (30) durch den Wärmetauscher (36) zu dem Absorber (40) zirkuliert.

6. Verfahren nach einem der vorherigen Ansprüche, wobei der Wärmetauscher (36) ein Zweistrom-Wärmetauscher (36) ist, das Verfahren umfassend das Mischen des Kopfstroms (72) und/oder des oberen Stroms (80), um den Abflussstrom (14) zu bilden, wobei der Kopfstrom (32) ausschließlich durch den Abflussstrom (14) zwischen dem Entspannungskolben (30) und dem Absorber (40) abgekühlt wird.

7. Verfahren nach einem der Ansprüche 1 bis 5, wobei der Wärmetauscher (36) ein Dreistrom-Wärmetauscher (36) ist, das Verfahren umfassend das Bilden eines ersten Abflussstroms aus dem Kopfstrom (72) und eines zweiten Abflussstroms aus dem oberen Strom (80), wobei der Kopfstrom (32) ausschließlich durch den ersten Abflussstrom und durch den zweiten Abflussstrom zwischen dem Entspannungskolben (30) und dem Absorber (40) abgekühlt wird.

8. Verfahren nach einem der vorherigen Ansprüche, wobei die Temperaturdifferenz zwischen dem abgekühlten Kopfstrom (58) und dem oberen Strom (32) als Absolutwert größer als 2 °C ist.

9. Verfahren nach einem der vorherigen Ansprüche, wobei der Strom (12) aus C2+-Kohlenwasserstoffen mehr als 90 Mol-% der C2-Kohlenwasserstoffe enthält, die in dem Reststrom (28) enthalten sind.

10. Verfahren nach einem der vorherigen Ansprüche, umfassend einen Vorreinigungsschritt und/oder aufeinanderfolgende Abkühlungs- und Trennschritte des Raffinerieabgases (16).

## Claims

1. Method for recovering a stream (12) of C2+ hydrocarbons in a residual refinery gas (16), comprising the following steps:
- forming a residual stream (28) from the residual refinery gas (16);
- feeding the residual stream (28) into a flash drum (30) to form a gaseous overhead flow (32) and liquid bottom flow (34);
- feeding the bottom flow (34) into a distillation column (38);
- cooling the overhead flow (32) in a heat exchanger (36) to form a cooled overhead flow (58);
- separating the cooled overhead flow (58) into a liquid lower stream (64) feeding the distillation column (38) and into a gaseous top stream (80);
- feeding the lower stream (64) into the distillation column (38) above the bottom flow (34);
- recovering the stream (12) of C2+ hydrocarbons at the bottom of the distillation column (38);
- extracting a gaseous overhead stream (72) at the head of the distillation column (38);
- forming at least one effluent stream (14) from the overhead stream (72) and/or from the top stream (80);
- heating the or each effluent stream (14) in a heat exchanger (36) via heat exchange with the overhead flow (32);
**characterized in that** the separation of the cooled overhead flow (58) comprises feeding the cooled overhead flow (58) into an absorber (40), and injecting a methane-rich stream (42) into the absorber (40) to place the cooled overhead flow (58) in contact with the methane-rich stream (42), the molar content of methane in the methane-rich stream (42) is higher than 90 mole %.

2. The method according to claim 1, wherein the methane-rich stream (42) is a liquid stream.

3. The method according to any of the preceding claims, wherein the methane-rich stream (42) is formed from a thermal steam cracking unit (63), without passing through the distillation column (38) or through the flash drum (30).

4. The method according to any of the preceding claims, wherein the temperature of the residual stream (28) before entering the flash drum (30) is lower than -80°C and is advantageously between -90 °C and -100°C.

5. The method according to any of the preceding claims, wherein the overhead flow (32) circulates from the flash drum (30) through the heat exchanger (36) as far as the absorber (40) without static or dynamic expansion in an expansion valve or dynamic expansion turbine.

6. The method according to any of the preceding claims, wherein the heat exchanger (36) is a two-flow heat exchanger (36), the method comprising the mixing of the overhead stream (72) and/or top stream (80) to form the effluent stream (14), the overhead flow (32) being cooled exclusively by the effluent stream (14) between the flash drum (30) and the absorber (40).

7. The method according to any of claims 1 to 5, wherein the heat exchanger (36) is a three-flow heat exchanger (36), the method comprising the formation of a first effluent stream from the overhead stream (72) and a second effluent stream from the top stream (80), the overhead flow (32) being cooled exclusively by the first effluent stream and by the second effluent stream between the flash drum (30) and the absorber (40).

8. The method according to any of the preceding claims, wherein the difference in temperature between the cooled overhead flow (58) and the overhead flow (32) is greater than 2°C in absolute value.

9. The method according to any of the preceding claims, wherein the stream (12) of C2+ hydrocarbons contains more than 90 mole % of the C2 hydrocarbons contained in the residual stream (28).

10. The method according to any of the preceding claims comprising a prior purification step and/or successive steps of cooling and separating the residual refinery gas (16).
